# EUROPEAN PATENT APPLICATION

(11) **EP 2 025 687 A1**
(43) Date of publication of application: **18.02.2009**
(21) Application number: 07112975.3
(22) Date of filing: 23.07.2007
(51) Int. Cl.: C08B 37/00, A61K 31/728, A61K 31/737

(54) **Process for the preparation of heparanase-inhibiting sulfated hyaluronates and products obtained thereby**

(71) Applicant: Istituto Scientifico di Chimica E Biochimica "G Ronzoni, 20133 Milano (IT); Technion's Rapport Family Institute for Research in Medical Sciences, Haifa (IL)
(72) Inventor: Naggi, Annamaria, Dr., 20025, Legnano (Mi) (IT); Torri, Giangiacomo, Dr., 20133, Milano (IT); Casu, Benito, 20133, Milano (IT); Vlodavsky, Israel, P.O. Box 9649 Haifa 31096 (IL)
(74) Representative: Serravalle, Marco

(57) **Abstract**

A series of heparanase-inhibiting, sulfated hyaluronates having an average molecular weight of from 6,000 to 38,000 and a degree of sulfation of from 1.8 to 4.0 were prepared from high-molecular weight hyaluronic acid by a method involving partial depolymerization and extensive O-sulfation, comprising a treatment of the starting hyaluronic acid with a sulphuric acid and chlorosulfonic acid mixture and optional size-fractionation, optionally followed by a selective 6-O-desulfation. Extensively sulfated hyaluronates (degree of sulfation of from 2.8 to 4) inhibit heparanase with at least the same potency as heparin. Subsequent O-desulfation affords new 6-O-desulfated, sulfated hyaluronates having a degree of sulfation of from 1.8 to 2.8 and also showing heparanase-inhibiting activity. Said O-sulphated hyaluronates are new glycosaminoglycans useful for the preparation of medicaments with antimetastatic and tumor growth inhibiting activity.

## Description

### FIELD OF INVENTION

The present invention refers to the preparation of new oversulfated hyaluronates and of their novel 6-O-desulfated analogs; and to the use thereof as antimetastatic agents. More particularly, the invention concerns heparanase-inhibiting, O-sulfated hyaluronates having an average molecular weight of from 6,000 to 38,000 Da prepared from high-molecular weight hyaluronic acid by a method involving partial depolymerization and concurrent extensive O-sulfation, said method comprising a treatment of the starting hyaluronic acid with a sulfuric acid/chlorosulfonic acid mixture at low temperature and optional size-fractionation, optionally followed by a 6-O-desulfation of the sulfated hyaluronate fractions and/or fragments thus obtained.

### BACKGROUND OF THE INVENTION

Heparanase is an endo-β-D-glucuronidase that cleaves the heparan sulfate chains of the heparan sulfate proteoglycans, which are ubiquitous components of the extracellular matrix and the surface of most animal cells. The active form of heparanase is a 58 kD protein containing an active site responsible for cleavage of glycosidic bonds of β-D-glucuronic acid residues of heparan sulfate chains and three basic clusters, two of which may be involved in tight binding of heparan sulfate [Hulett MD et al. Biochemistry 2000;39:15659-15667 - Levy-Adam F et al. J Biol Chem 2005;280;1203-1213]. Most cancer cells overexpress the enzyme, which causes release in active form of growth factors stored by heparan sulfate proteoglycans (angiogenic effect), as well as disruption of basement membranes and extravasation of tumor cells (metastatic effect) [Vlodavsky I, et al., J Clin Inv 2001;108:341-347]. Heparanase promotes also tumor growth. Inhibition of heparanase is therefore an attractive target for development of antitumor drugs.

One of the strategies for inhibiting heparanase involves hindering of the binding to the enzyme of heparan sulfate chains of heparan sulfate proteoglycans, among others by using heparan sulfate mimics likely competing with heparan sulfate proteoglycans for the basic clusters of heparanase involved in binding and recognition of heparan sulfate chains [Ishai-Michaeli Ret al. Biochemistry 1992;31:2080-2088 - Parish CR, Freeman C et al. Cancer Res 1999; 59:3433-3441]

Inhibitors of the enzyme heparanase constitute an interesting target in antimetastatic cancer therapy.

In the field of products endowed with heparanase-inhibition properties, mimicking heparan sulfate, literature discloses a large number of sulfated polysaccharides and corresponding oligosaccharide fragments.

Heparin, an animal polysaccharide belonging to the glycosaminoglycan family, is one of the closest mimics of heparan sulfate [Ferro V et al., Mini Reviews Med Chem 2004;4:693-702] and actually is a potent inhibitor of the enzyme [Casu B et al. Adv Carb Chem Biochem 2001;57:159-206]. However, the strong anticoagulant activity of this glycosaminoglycan, mainly associated with specific pentasaccharide sequences that constitute the active site for antithrombin, makes this drug unsuitable for long-term pharmacological treatments.

"Glycol-split" heparins, i.e. periodate oxidized and desulfated, periodate oxidized heparin where all nonsulfated uronic acid residues (including glucuronic acid residues of the active site for antithrombin) are drastically modified, have very low anticoagulant activity and were shown to be antiangiogenics [WO] and heparanase inhibitors [WO - Naggi A et al. J Biol Chem 2005;280;12103-12113] while no longer acting as substrates for the enzyme. However, glycol-split heparins still retain some of the intrinsic structural heteogeneities of the original heparin structure.

Also laminaran, the polyglucose natural precursor of the potent heparanase inhibitor and antimetastatic agent laminaran sulfate [Miao H-Q et al., Int J Cancer 1999;83, 424-43], is structurally non homogeneous because of occasional branching that occurs to different extents in different preparations [Naggi A et al, unpublished results].

Two chemically fully sulfated oligosaccharides, i.e. maltohexaose sulfate ("MHS"), and phosphomannopentaose sulfate ("PI-88") also showed to be excellent heparanase inhibitors [Parish CR, Freeman C et al. Cancer Res 1999; 59:3433-3441]. However, their structure is largely different from that of heparan sulfate, so that they cannot be considered as close mimics of the natural target of heparanase.

### DESCRIPTION OF THE PRIOR ART

Hyaluronic acid is a linear glycosaminoglycan formed by a mixture of chains of different length consisting of repeating sequences of the glucuronyl-β-1→3-N-acetylglucosamine disaccharide unit and, therefore, each chain in said mixture of chains shows the same repetitive sequence of formula (A) 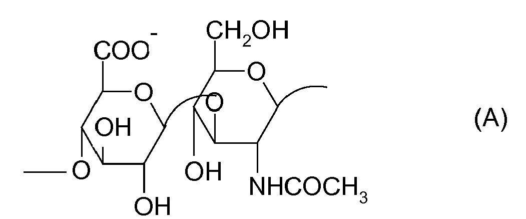 the corresponding cation generally being hydrogen (hyaluronic acid) or sodium (sodium hyaluronate). The chains forming natural hyaluronic acid are very long, such that the average molecular weight of the mixture of chains is of the order of 10⁶ Da.

Herein below, by convention, the term "hyaluronic acid" globally designates the native product while the term "sulfated hyaluronates" globally designates the differently O-sulfated hyaluronic acid derivatives, generally in form of a salt thereof, especially as sodium salt, those having a degree of sulfation of at least 2.8 being referred to as "oversulfated hyaluronates".

Furthermore, by convention, the positions of the sulfo (SO₃⁻) groups introduced on the free hydroxyls of hyaluronic acid are herein indicated as follows: "6-O-sulfate (6S)" refers to the sulfo group on the primary hydroxyl of the glucosamine; "4-O-sulfate (4S)" refers to the sulfo group on the 4-hydroxyl of glucosamine; "2-O-sulfate (2S)", instead of 2'-O-sulfate, refers to the sulfo group on the 2-hydroxyl of the glucuronic acid and "3-O-sulfate (3S)", instead of 3'-O-sulfate, refers to the sulfo group on the 3-hydroxyl of the glucuronic acid. This is possible because there is no hydroxy group in the 2 and 3 positions of glucosamine and because the concurrent possible 100% sulfation of the 4' hydroxyl of the non-reducing end of each chain in said mixture of chains does not have any appreciable influence on the degree of sulfation of the final O-sulfated hyaluronates.

Finally, the glucosamine residue may be referred to as "A" while the glucuronic acid residue may be referred to as "G", for example A4-OH, A6-OH or G2S(30H) to designate the 4-hydroxyl of the glucosamine, the 6-hydroxyl of the glucosamine and the 2-sulfo group of glucuronic acid with a free 3-hydroxyl, respectively.

DE 19813234 discloses oversulfated hyaluronates with a degree of sulfation of more than 3.5 and a molecular weight of from 60,000 to 300,000, prepared by sulfation of an ammonium hyaluronate with the sulfur trioxide/dimethylformamide complex. These highly sulfated hyaluronates are described as profibrinolytic or anticoagulant activity useful for treating thrombophlebitis For these products, a hyaluronidase-inhibiting activity is also described.

WO 95/25751 discloses hyaluronic acid, hyaluronate esters and salts thereof which are sulfated in order to obtain derivatives with a degree of sulfation of from 0.5 to 3.5 exhibiting anticoagulant activity and cell adhesion reduction properties and useful to prepare biomaterials. According to this document, the sulfated hyaluronates are prepared by reacting the tetrabutylammonium salt of hyaluronic acid with pyridine.SO₃. In particular, this document cites the possibility to obtain a sulfated hyaluronate fraction having a molecular weight in the range between about 10,000 and about 50,000 and a degree of sulfation of 2.5, 3 or 3.5, prepared by sulfation of fractions obtained by the use of membranes with suitable molecular weight cut-off. However the document neither discloses nor, *a fortiori*, identifies said sulfated fraction.

US 5,872,109 discloses sulfated hyaluronates as anti-inflammatory agents for the treatment of respiratory distress syndrome. Said hyaluronates, which are described as having a molecular weight of 40,000 or more are prepared by reaction of hyaluronic acid with sulfuric acid/trimethylamine complex. This document states that low molecular weight hyaluronic acid may be prepared by partial hydrolysis of hyaluronic acid having a higher molecular weight, but it does not describes such a partial hydrolysis. In addition, US 5,872,109 discloses the sulfation of a hyaluronic acid having a molecular weight of 40,000 giving a hyaluronate sulfated at 50-60%, which, in the absence of any other indication, should have a molecular weight of at least 55,000.

Analogously, US 5,008,253 discloses previously depolymerized hyaluronic acid having a molecular weight of 14,000 which is N-deacetylated, N-sulfated and subsequently selectively O-sulfated using trimethylamine.sulfur trioxide adduct as a sulfating agent to give a N-deacetyl-N-sulfohyluronate-6-O-sulfate having anti-arteriosclerotic activity with reduced anticoagulant activity but it does not describes how said depolymerized hyaluronic acid has been obtained.

US 5,491,227 describes a method for the controlled molecular weight reduction of hyaluronic acid by pressure homogenization, i.e. by passing a solution of hyaluronic acid through a pressure homogenizer, in order to obtain a hyaluronic acid having a molecular weight of from 1.2 to 1.5x10⁶ Da. The minimal molecular weight described for reduced molecular weight hyaluronic acid is of 84 kD after 20 passages through the pressure homogenizer.

JP 2001-097997 discloses sulfated hyaluronates having a molecular weight of from 10,000 to 1,500,000 and a sulfation degree of from 0.5 to 4 for use as carriers for affinity absorption capable of fractionally adsorbing proteins.

WO 2005/046562 discloses the use of sulfated hyaluronates for inhibiting or treating inflammatory states of the eye, in particular blepharitis and conjunctivitis. The used sulfated hyaluronates are prepared according to the above-cited DE 19813234, the lowest low molecular weight sulfate hyaluronate described having a molecular weight of 150,000.

EP 116251 discloses a process for the depolymerization and the sulfation of polysaccharides by reaction of the starting polysaccharides with a mixture of sulfuric acid and chlorosulfonic acid, said process being carried out at a temperature of -20°C to +40°C. According to this document, when the reaction with sulfuric acid/chlorosulfonic acid is made on polysaccharides already containing sulfate groups, such as heparin, chorndoitinsulfate or dermatansulfate, said reaction leads to a concurrent depolymerization and oversulfation affording depolymerized and oversulfated polysaccharides. On the contrary, according the same document, the action of the sulfuric acid/chlorosulfonic acid mixture, at 0-4°C or at room temperature, on a non-sulfated polysaccharide such as chitosan or chitin, regularly leads to polysaccharides having a sulfation degree per monosaccharidic units of 1, i.e. to 6-O-sulfated polysaccharides. This could be the reason of the use of tertiary amines/sulfur trioxide adducts for the sulfation of hyaluronic acid in order to obtain sulfated hyaluronates.

### SUMMARY OF THE INVENTION

Hyaluronic acid is the most structurally homogeneous among the glycosaminoglycans of animal origin [Fransson L-Å. Glycosaminoglycans. In: The polysaccharides (Aspinall GO, ed), Academic Press, Orlando, FL, 1985;3:337-413]. In fact, as set forth above, it is exclusively made up of nonsulfated glucuronic acid and acetylglucosamine residues (formula A above).

The present invention is based on the hypothesis that, since the glucuronic acid residues of hyaluronic acid are linked to glucosamine *via* β1,3 (instead of β1,4 as in heparan sulfate and heparin), this glycosaminoglycan should not be cleaved by heparanase even upon appropriate sulfation. Furthermore, according to this hypothesis, the structural homogeneity of hyaluronic acid should be a favorable condition to obtain polysulfated derivatives as putative inhibitors of heparanase.

The present invention is also based on the hypothesis that sulfated hyaluronates having a relatively low average molecular weight, possibly not higher than 40,000-50,000, and a degree of sulfation of at least 1.8 could be good heparanase inhibitors useful to prepare antimetastatic and tumor growth inhibiting medicaments.

However, even though literature discloses a number of sulfated hyaluronic acids and cites low molecular weight hyaluronate sulfates, actually sulfate hyaluronates having a sulfation degree of from 1.8 to 4 and an average molecular weight not higher than 40,000-50,000 are not disclosed in the literature. Moreover, the general method used for their preparation substantially is the classical O-sulfation with a tertiary amine.SO₃ adduct or, for very high sulfation degrees, the method disclosed in DE 19813234, using a dimethylformamide.SO₃ adduct.

It has now been found that hyaluronic acid behaves differently from the other unsulfated polysaccharides in respect of the reaction with the sulfuric acid/chlorosulfonic acid mixture described in EP 116251. In fact, contrary to its unsulfated congeners (such as chitosan, or chitin), which only receive one SO₃⁻ group (normally in 6-position) per disaccharide unit by operating at both low and room temperature, hyaluronic acid is concurrently depolymerized and oversulfated if the reaction with the sulfuric acid/chlorosulfonic acid mixture is carried out at a temperature of from -15 to +5°C while it is destroyed if the reaction is carried out at room temperature.

It has also been found that, by submitting hyaluronic acid to the action of a sulfuric acid/chlorosulfonic acid mixture at a temperature of from -15 to +5°C, oversulfated hyaluronates having a molecular weight of from 8,000 to 38,000 and a sulfation degree of from 2.8 to 4.0 are obtained. The oversulfated hyaluronates thus obtained may be size-fractionated in order to obtain fractions having a more homogeneous molecular weight range and a degree of sulfation of from 2.8 to 4.0, preferably from 2.8 to 3.8.

Moreover, it has been found, that, by submitting the sulfated hyaluronate fractions and/or fragment thus obtained to a selective 6-O-desulfation, novel 6-O-desulfated, sulfated hyaluronate fractions and/or fragments having a molecular weight of from 6,000 to 30,000 and a sulfation degree of from 1.8 to 3 are prepared.

Finally, it has been found that, all the sulfated hyaluronate fractions and/or fragments having a molecular weight of from 6,000 to 38,000 and a sulfation degree of from 1.8 to 4.0, preferably from 1.8 to 3.8 exhibit a remakble heparanase inhibiting activity which renders said product useful to prepare medicaments for the prevention and treatment of metastases or for the inhibition of the growth of tumors, such as myeloma.

In particular, the O-oversulfated hyaluronates and their fractions, having an average molecular weight of from 8,000 to 38,000 and a degree of sulfation of from 2.8 to 4.0, preferably from 2.8 to 3.8, exhibit a heparanase inhibiting activity higher than that of heparin while their anticoagulant activity is much lower than that of heparin and also show antimetastatic activity *in vivo*.

In addition, the 6-O-desulfated, sulfated hyaluronate fractions and/or fragments having a molecular weight of from 6,000 to 30,000 and a sulfation degree of from 1.8 to 3 preferably 1.8 to 2.8 still have a good heparanase activity and are very good antimetastatic agents. In particular, these O-sulfated hyaluronates free of 6-O-sulfo groups show a heparanase-inhibiting activity similar to that of heparin but exhibit a much lower anticoagulant activity than that of heparin. They also show antimetastatic activity *in vivo*.

The expression "sulfated hyaluronate fractions and/or fragments", as used herein, globally indicates (i) O-sulfated glycosaminoglycans obtained by sulfuric acid/chlorosulfonic acid fragmenting depolymerization of natural hyaluronic acid with concurrent O-sulfation, and their 6-O-desulfo derivatives; (ii) O-sulfated glycosaminoglycans obtained by sulfuric acid/chlorosulfonic acid fragmenting depolymerization of natural hyaluronic acid with concurrent O-sulfation, followed by fractionation, and their 6-O-desulfo derivatives.

### BRIEF DESCRIPTION OF THE DRAWINGS

Figure 1 shows the heteronuclear single quantum coherence (HSQC) spectra of sample G4702 (Example 1) and of G5914 (Comparative Example 8).
Figure 2 shows the heteronuclear single quantum coherence (HSQC) spectrum of sample G4810 (Example 7).

### DETAILED DESCRIPTION OF THE INVENTION

It is an object of the present invention to provide a process for the preparation of a glycosaminoglycan consisting of sulfated hyaluronate fractions and/or fragments having a sulfation degree of from 6,000 to 38,000 Da and a sulfation degree of from 1.8 to 4 which comprises
(a) treating hyaluronic acid with a sulfuric acid/chlorosulfonic acid mixture at a temperature of from -15°C to +5°C and optionally submitting the product thus obtained to a size-fractionation, whereby oversulfated hyaluronate fractions and/or fragments are obtained, having an average molecular weight of from 8,000 to 38,000 Da, consisting of a mixture of chains of different length in which each chain in said mixture of chains shows the repetitive sequence of formula I 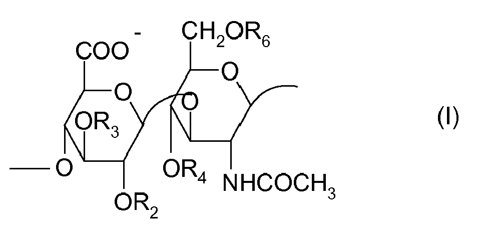 wherein each of R₂, R₃, R₄ and R₆ represents hydrogen or a SO₃⁻ group, for a sulfation degree of from 2.8 to 4.0, preferably from 2.8 to 3.8; and, optionally,
(b) treating the tertiary amine or quaternary ammonium salt of the oversulfated hyaluronate fractions and/or fragments mixture thus obtained with an silylating agent, at 45-110°C, whereby sulfated hyaluronate fractions and/or fragments having an average molecular weight of from 6,000 to 30,000 Da, consisting of a mixture of chains in which at least 90% of said chain show the repetitive sequence of formula I' 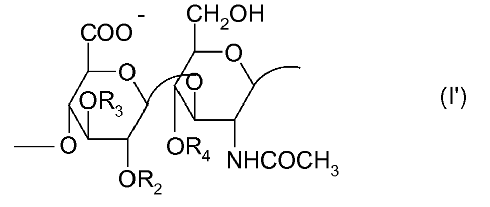 wherein each of R₂, R₃ and R₄ represents hydrogen or a SO₃⁻ group, for a sulfation degree of from 1.8 to 3, preferably from 1.8 to 2.8, are obtained.

It is another object of the present invention to provide new glycosaminoglycans consisting of sulfated hyaluronate fractions and/or fragments, obtainable according to this process, having an average molecular weight of from 6,000 to 38,000 Da and a sulfation degree of from 1.8 to 4, preferably from 1.8 to 3.8, and salt thereof. These products are heparanase inhibiting, and tumor growth inhibiting antimetastatic agents.

Any commercially available hyaluronic acid, preferably in form of its sodium salt, may be used as starting material of the process of the present invention. Said starting sodium hyaluronate will advantageously have an average molecular weight of from 100,000 to 1,500,000, preferably from 150,000 to 800.000 Da.

Step (a), consisting of a concurrent depolymerization and O-sulfation, is carried out by reacting the starting hyaluronic acid in an appropriate mixture of H₂SO₄ and HClSO₃. Typically, the starting hyaluronic acid, preferably as sodium salt, is added, under gentle stirring, to a mixture formed by 98% sulfuric acid and by chlorosulfonic acid at a temperature of from -15 to +5°C, preferably at -4/+5°C. The H₂SO₄/HClSO₃ ratio may vary from 3/1 to 1.5/1. After the addition, the reaction mixture is let to stand under stirring at the same temperature for 20-120 minutes. At the end of the reaction the depolymerized and oversulfated hyaluronate thus obtained is isolated by pouring the reaction mixture into a cold organic solvent such as ethyl ether, filtering the precipitate, dissolving it in a NaHCO₃ solution and removing the salts.

By controlling the water contents of the H₂SO₄/HClSO₃ mixture, it is possible to have reproducible results in terms of degree of sulfation and molecular weight. In addition, by suitably choosing the appropriate H₂SO₄/ HClSO₃ ratio, temperature and sulfation duration, a depolymerized and oversulfated hyaluronate having an average molecular weight of from 12,000 to 38,000 Da and a degree of sulfation of from 2.8 to 4.0, advantageously from 2.8 to 3.8, preferably from 3.4 to 3.8, is normally obtained at the end of the reaction.

The depolymerized and oversulfated hyaluronate obtained at the end of step (a) consists of a mixture of chain of different length in which each chain in said mixture of chains shows the repetitive sequence of formula I, wherein each of R₂, R₃, R₄ and R₆ may be a SO₃⁻ group in more than 95% of said chains, for a degree of sulfation, calculated by heteronuclear 2D NMR spectra using the ratio between appropriate signals volumes as indicated in Guerrini et al. publication (see the introduction to the Examples), higher than 3.5. However, by lessening the reaction time or the temperature toward -15°C, or both, a depolymerized and oversulfated hyaluronate showing the repetitive sequence of formula I wherein R₆ is a SO₃⁻ group in more than 95% of said chains and each of R₂, R₃, and R₄ may be a SO₃⁻ group in from 70 to 100% of said chains, for a sulfation degree of from 2.8 to 3.5 may be obtained.

The depolymerized and oversulfated hyaluronate having a molecular weight of from 12,000 to 38,000 Da, obtained at the end of the reaction of hyaluronic acid with the H₂SO₄/HClSO₃ mixture may be submitted to a size-exclusion fractionation in order to obtain depolymerized and O-oversulfated hyaluronate fractions having more homogeneous molecular weight distributions with an average molecular weight of from 6,000 to 30,000.

Thus, at the end of step (a) including the optional fractionation there are obtained oversulfated hyaluronate fractions and/or fragments which are generally isolated as sodium salts. Said hyaluronates may be converted into other salts which can be used for chemical syntheses ("chemically acceptable salts") or as active ingredients for the preparation of pharmaceutical compositions for combating metastases ("pharmaceutically acceptable salts"). Advantageous salts are the alkaline metal, alkaline-earth metal, tertiary amine, quaternary ammonium, aluminum and zinc salts. Preferred salts are the sodium, potassium, calcium, magnesium, pyridinium, tetra(n-butyl) ammonium, tri (n-butyl) amine, aluminum and copper salts.

The oversulfated hyaluronate fractions and/or fragments obtained at the end of step (a) are heparanase inhibiting antimetastatic agents which, contrary to the oversulfated hyaluronates described for example in WO 95/25751 or in DE 19813234, have an anticoagulant activity which is lower than that of heparin and low molecular weight heparin.

In the optional step (b), the oversulfated hyaluronate fractions and/or fragments mixture obtained at the end of step (a), preferably as sodium salt, is submitted to partial O-desulfation to obtain novel 6-O-desulfated, sulfated hyaluronates. The 6-O-desulfation may occur by treatment of a tertiary amine or quaternary ammonium salt of the oversulfated hyaluronate fractions and/or fragments mixture obtained at the end of step (a) with a silylating agent. To this purpose, said oversulfated hyaluronate fractions and/or fragments are converted into a tertiary amine or quaternary ammonium salt thereof by neutralizing an aqueous solution thereof, for example by using a ion exchange resin in H⁺ form, and treating the oversulfated hyaluronic free acid mixture thus obtained with a tertiary amine or a quaternary ammonium hydroxide, for example pyridine or tetra(*n*-butyl)ammoniun hydroxide. Typically, an aqueous solution of the oversulfated hyaluronate fractions and/or fragments mixture is poured onto a column containing the acidic resin, for example Amberlite^{®} IR-120 in the H⁺ form and, after washing the resin, the eluate is brought to pH 7- 9 with pyridine, the solution is concentrated and freeze dried. The pyridine salt thus obtained may be dissolved in pyridine may by treated with N,O-bis-(trimethylsilyl)acetamide, or N-methyl-N-(trimethylsilyl)trifluoroacetamide and the solution is kept at 45-110°C for 2-4 hours. At the end of the reaction, novel, 6-O-desulfated O-sulfated hyaluronates having an average molecular weight of from 6,000 to 30,000 Da, consisting of a mixture of chains in which at least 90% of said chains show the repetitive sequence of formula I' above, wherein each of R₂, R₃ and R₄ represents hydrogen or a SO₃⁻ group, for a sulfation degree of from 1.8 to 3, is obtained. The obtained 6-O-desulfatedsulfated hyaluronate fractions and/or fragments mixture is isolated, generally as sodium salts, according to conventional techniques, for example by addition of water to the mixture, neutralization with a base (for example sodium hydroxide) and concentrated to dryness to give, after desalting by size exclusion fractionation, a substantially 6-O-desulfated, O-sulfated hyaluronate.

The substantially 6-O-desulfated O-sulfated hyaluronate is generally isolated as sodium salt. Said sodium salt may be converted into other salts which can be used for chemical syntheses or as active ingredients for the preparation of pharmaceutical compositions for combating metastases. Advantageous salts are the alkaline metal, alkaline-earth metal, tertiary amine, quaternary ammonium, aluminum and zinc. Preferred salts are the sodium, potassium, calcium, magnesium, pyridinium tetra(n-butyl)ammonium and copper salts. Said 6-O-desulfated, sulfated hyaluronate fractions and/or fragments mixtures are new glycosaminoglycans.

It is a further object of the present invention to provide a glycosaminoglycan consisting of sulfated hyaluronate fractions and/or fragments having an average molecular weight of from 6,000 to 30,000 Da, consisting of a mixture of chains in which at least 90% of said chain show the repetitive sequence of formula I' above, wherein each of R₂, R₃ and R₄ represents hydrogen or a SO₃⁻ group, for a sulfation degree of from 1.8 to 3, preferably from 1.8 to 2.8 and their salts.

These new glycosaminoglycans have a good heparanase-inhibiting activity, of the same order as that of heparin, with a low anticoagulant activity and are useful for preparing medicaments for combating metastases and inhibiting tumor growth.

Thus, the process of the present invention allows the preparation of new glycosaminoglycans consisting of sulfated hyaluronate fractions and/or fragment having an average molecular weight of from 6,000 to 38,000 and a sulfation degree of from 1.8 to 4, and the salts thereof, which are heparanase-inhibiting agents having antimetastatic and tumor growth in inhibiting activity useful for the preparation of pharmaceutical compositions.

The sulfated hyaluronates for use as active ingredients for the preparation of medicament having antimetastatic and tumor growth inhibiting action were tested for their ability to inhibit heparanase using metabolically sulfate-labeled ECM as a substrate [Bar-Ner, et al. (1987), Blood 70, 551-557; Vlodavsky, I. Et al. (1983) Cancer Res. 43, 2704-2711]. Sulfate-labeled ECM coating the surface of 35-mm culture dishes was incubated (4 h, 37°C, pH 6.0) with recombinant human heparanase (40 ng/ml) in the absence and presence of three concentrations (0.2, 1.0, 5.0 µg/ml) of each hyaluronate species. The reaction mixture contained 50 mM NaCl, 1 mM dithiothreitol, 1 mM CaCl₂, and 10 mM phosphate-citrate buffer, pH 6.0. To evaluate the occurrence of proteoglycan degradation, the incubation medium was collected and applied for gel filtration on Sepharose 6B columns (0.9 x 30 cm). Fractions (0.2 ml) were eluted with PBS at a flow rate of 5 ml/h and counted for radioactivity. The excluded volume (Vo) was marked by blue dextran, and the total included volume (Vt) was marked by phenol red. Nearly intact heparin sulfate proteoglycans are eluted from Sepharose 6B just after the void volume (Kav < 0.2, fractions 1-10), while heparin sulfate degradation fragments are eluted toward the Vt of the column (peak II, 0.5 < Kav< 0.8, fractions 15-35) [Vlodavsky, I. Et al. (1999) Nat. Med. 5, 793-802]. Labeled fragments eluted in peak II were shown to be degradation products of heparan sulfate as they were 5-6-fold smaller than intact heparan sulfate chains of heparin sulfate proteoglycans, resistant to further digestion with papain and chondroitinase ABC, and susceptible to deamination by nitrous acid. Heparanase activity = Kav x total cpm in peak II. Recovery of labeled material applied on the column ranged from 85 to 95% in different experiments. Each experiment was performed at least three times, and the variation in elution positions (Kav values) did not exceed ±15%.

The tested glycosaminoglycans consisting of sulfated hyaluronate fractions and/or fragments as illustrated above have shown a very high heparanase-inhibiting activity, even higher than that of heparin and are thus useful for preventing and treating metastases as well as for inhibiting tumor growth, for example in myeloma.

The in vivo antimetastatic activity of these sulfated hyaluronates having a degree of sulfation of from 1,8 to 4.0 has been tested in mice according to the method of experimental melanoma metastasis described by I. Vlodavsky, M. Mohsen, O. Lider, C.M. Svahn, H.P. Ekre, M. Rigoda, R. Ishai-Michaeli , T. Peretz "Proteases and their inhibitors in invasion and metastasis", Invasion Metastasis 1994-95; 14:290-302 (see also I. Vlodavsky and M. Elkin : "Tail vein assay of cancer metastasis". Current Protocols in Cell Biology. John Wiley & Sons, New York, N.Y., 2001, pp 19.2.1-19.2.7) . In a test, C57BL mice (female, 7-8 week old, 5 mice per group) received a single intraperitoneal injection of 0.2 ml phosphate buffer solution (PBS), as a control, or 0.2 ml PBS containing 300 µg of each of the products to be tested, 15-20 minutes prior to an intravenous inoculation of B16-BL6 melanoma cells (1 x 105 cells/mouse), suspended in culture medium. Mice were sacrificed 15 days later, the lungs fixed in Bouin's solution (consisting of 750 ml picric acid, saturated aqueous solution; 250 ml 37-40% aqueous formaldehyde; 50 ml glacial acetic acid) and scored for the number of metastatic melanoma nodules on the lung surface. An average number of lesion of about 110 (Standard Deviation = 50) was observed in control mice while an average number of 6-16 lesions (Standard Deviation = 3.3 - 6.6) appeared on the lung surface of mice previously treated with the sulfated hyaluronates of the present invention.

Thus it is a further object of the present invention to provide the use of glycosaminoglycans consisting of sulfated hyaluronate fractions and/or fragments as illustrated above, having a mean molecular weight of from 6,000 to 38,000 and a degree of sulfation of from 1.8 to 4, advantageously from 2.8 to 4, preferably from 2.8 to 3.8, and their pharmaceutically acceptable salts preferably alkaline metal, or alkaline earth metal for the preparation of medicaments with antimetastatic and tumor growth inhibiting activity.

According to an advantageous embodiment, said glycosaminoglycans are sulfated hyaluronate fractions and/or fragments obtainable by the above-illustrated oversulfation process using a sulfuric acid/chlorosulfonic acid mixture at a temperature of from -15°C to +5°C, optional followed by a 6-O-desulfation performed by using silyl derivatives.

According to another advantageous embodiment, said glycosaminoglycans are sulfated hyaluronic acid fractions and/or fragments having an average molecular weight of from 8,000 to 38,000 Da and consisting of mixtures of chains of different length in which each chain in said mixtures of chains shows the repetitive sequence of formula I above, wherein each of R₂, R₃, R₄ and R₆ represents hydrogen or a SO₃⁻ group for a sulfation degree of from 2.0 to 4.0, preferably from 2.8 to 3.8.

According to a further advantageous embodiment, said glycosaminoglycans are sulfated hyaluronic acid fractions and/or fragments having an average molecular weight of from 6,000 to 30,000 Da and consist of mixtures of chains of different length in which at least 90% of said chains show the repetitive sequence of formula I' wherein each of R₂, R₃ and R₄ represents hydrogen or a SO₃⁻ group, for a sulfation degree of from 1.8 to 3, preferably from 1.8 to 2.8.

In order to prepare medicaments with antimetastatic and tumor growth inhibiting activity, the above illustrated heparanase inhibiting glycosaminoglycans obtained according to the process of the present invention, in particular the sulfated hyaluronate fractions and/or fragments having a sulfation degree of from 6,000 to 38,000 Da having the structures (I) and (I') above, and their pharmaceutical acceptable salts, are formulated with conventional carriers or vehicles in pharmaceutical compositions which represent a further object of the present invention. In said pharmaceutical compositions for oral, subcutaneous, intravenous, transdermic or topic administration, the above illustrated heparanase inhibiting, sulfated hyaluronates active ingredients are preferably administered as dosage units, in admixture with the classic carriers or pharmaceutical vehicles. The dose can amply change in function of age, weight, and health conditions of the patient, as much as of severity of the disease and of route of administration. This dose comprises the administration once to three times per day at a dosage of 0.1 to 1,000 mg/kg, advantageously from 1 to 100 mg/kg, preferably from 2.5 to 50 mg/kg by intravenous (for example in infusion), intramuscular, subcutaneous, intraperitoneal, intranasal, enteral route or locally on the diseased tissue after surgical operation. Advantageously, the treatment may be made prior (2-3 days) and after (2-3 weeks) surgical removal of the primary tumor, thus being beneficial in inhibiting metastatic spread after surgical removal of the tumor by a possible exposure of tumor cells to the blood circulation. Treatment at this time, possibly by simply rinsing the affected area with the glycosaminoglycan of the present invention, may be beneficial, followed by a daily administration (once or twice) during the next 2-3 weeks. Inhibition of the growth of tumors, such as myeloma, may also be attained by treating patients with the glycosaminoglycans of the present invention.

The following examples illustrate the invention without, however, limiting it. All the final products were isolated as sodium salt thereof.

Molecular weight determinations were performed by GPC-HPLC on a Viscotex instrument equipped with a VE1121 pump, Rheodyne valve (100 µl), and triple detector array 302 equipped with IR, viscosimeter, and 90° light-scattering systems. Two 300 x 7.8-mm TSK GMPWXL Viscotek columns were used with 0.1 M NaNO₃ as eluent (flow, 0.6 ml/min). Samples were dissolved in the eluent solution at the concentration of 15 mg/ml. The average molecular mass was determined by size exclusion chromatography with a triple detector array.

NMR spectra were recorded at 500 MHz for ¹H and 125 MHz for ¹³C with a Bruker AMX spectrometer equipped with a 5-mm ¹H/X inverse probe. The spectra were obtained at 45°C from D₂O solutions (15 mg/0.5 ml D₂O, 99.99% D). Chemical shifts, given in parts per million down field from sodium-3-(trimethylsilyl) propionate, were measured indirectly with reference to acetone in D₂O (δ 2.235 for ¹H and δ 30.20 for ¹³C). The ¹³C NMR spectra were recorded at 300 or 400 MHz with a Bruker AC-300 or AMX-400 spectrometer.

The degree of sulfation/sulfated group distribution was calculated by heteronuclear single quantum coherence (HSQC) NMR spectrum determination of sulfate residues (ratio between the volumes of signals of each position sulfated and not sulfated, i.e. 6S/60H; 4S/4OH; G2S/G+G2S+G2,3S; G3S/G+G2S+G2,3S) , as suggest by Guerrini et al. in Analytical Biochemistry 337, 35-47, 2005.

### Example 1

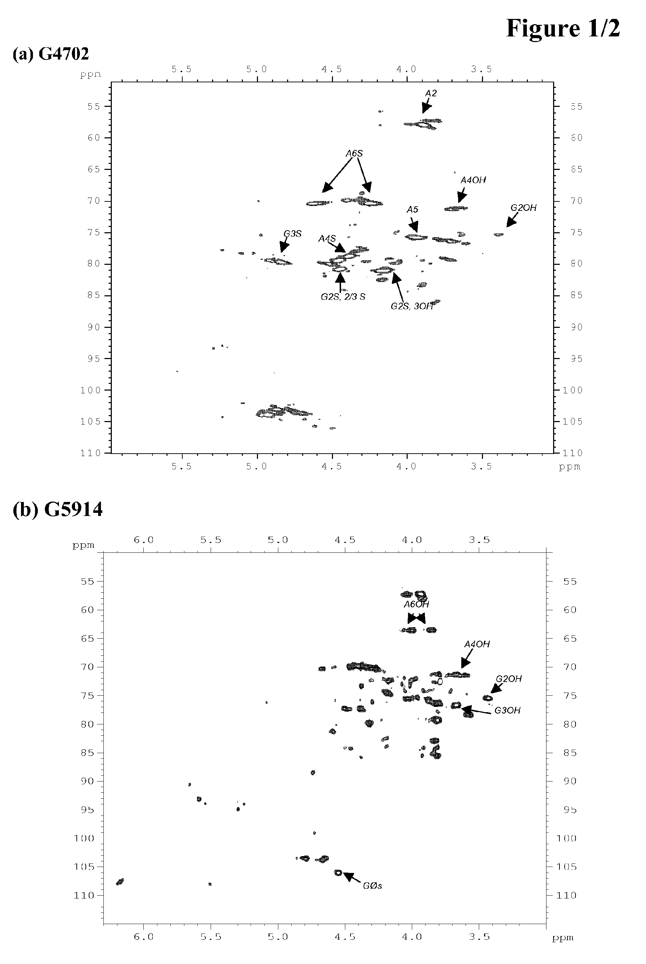

To a mixture of sulfuric acid and chlorosulfonic acid, obtained by adding 5 ml of chlorosulfonic acid to 10 ml of 98% sulfuric acid, previously cooled on a ice bath and stirred until the development of foam ceased, 500 mg of hyaluronic acid acid sodium salt having a mean molecular weight of 626.000 Da (HA-1) were added in small portions in about 15 minutes, then the mixture was let to stand at 4°C for 30 minutes under stirring. At the end of the reaction the mixture was poured into 300 ml of ethyl ether cooled with liquid nitrogen and the solid was filtered, washed with ethyl ether thus obtaining a greenish powder which was taken up with water. The solution was neutralized with 0.1 M sodium hydroxide and concentrated to dryness with rotavapor to give, after desalting by size exclusion fractionation on a 5 x 67 cm TSK 40S column using 10% ethanol in water as eluent and UV detection at 210 nm. (flow 5,4 ml/min), 442 mg of depolymerized, oversulfated hyaluronate having an average molecular weight of 36,000 Da (SHA-1). Its HSQC NMR spectrum only shows minor signals of free 4-OH glucosamine (20%) and 6-OH glucosamine (18%) and glucuronic acid 2-sulfate (30H) (12%). The sulfation pattern, determined according to Guerrini et al. Analytical Biochemistry 337 (2005) 35-47 is shown in Figure 1(a), for a degree of sulfation of 3.5-3.8.

In the test of heparanase inhibition described above (three experiments) this oversulfated hyaluronate gave a mean inhibition of 87.4% at a concentration of 1 µg/ml and of 92.8% at a concentration of 5 µg/ml, against a 79.7% inhibition at 1 µg/ml and 81.3% at 5 µg/ml obtained with heparin and 63.6% inhibition at 1 µg/ml and 83.4% at 5 µg/ml obtained with maltohexaose polysulfate.

### Example 2

To a mixture of sulfuric acid and chlorosulfonic acid, obtained by adding 2.8 ml of chlorosulfonic acid to 5.6 ml of 98% sulfuric acid, previously cooled on a ice bath and stirred until the development of foam ceased, 280 mg of hyaluronic acid sodium salt (HA-1) were added in small portions in about 20 minutes, then the mixture was let to stand at 4°C for 30 minutes under stirring. At the end of the reaction the mixture was poured into 100 ml of ethyl ether cooled with liquid nitrogen and the solid was filtered, washed with ethyl ether thus obtaining a greenish powder which was taken up with water. The solution was neutralized with a satured solution of sodium bicarbonate and concentrated to dryness with rotavapor to give, after desalting by dialysis against water with 2000-Da cut-off membranes 230 mg of depolymerized, oversulfated hyaluronate having an average molecular weight of 29000 Da (SHA-2). Its HSQC spectrum only shows minor signals of free glucosamine 4-OH (20%), 6-OH (13%), glucuronic acid 2-OH (11%) and glucuronic acid 2-Sulfate (30H) (4%) for a degree of sulfation higher than 3.4 and lower than 4.

### Example 3

To a mixture of sulfuric acid and chlorosulfonic acid, obtained by adding 2.8 ml of chlorosulfonic acid to 5.6 ml of 98% sulfuric acid, previously cooled on a ice bath and stirred until the development of foam ceased, 280 mg of hyaluronic acid sodium salt having an average molecular weight of 187,000 (HA-2) were added in small portions in about 20 minutes, then the mixture was let to stand at 4°C for 60 minutes under stirring. At the end of the reaction the mixture was poured into 100 ml of ethyl ether cooled with liquid nitrogen and the solid was filtered, washed with ethyl ether thus obtaining a greenish powder which was taken up with water. The solution was neutralized with a saturated solution of sodium bicarbonate and concentrated to dryness with rotavapor to give, after desalting by dialysis against water with 2000-Da cut-off membranes, 230 mg of depolymerized, oversulfated hyaluronate having an average molecular weight of 15,800 Da and a sulfation degree of 3.4-3.6 (SHA-3).

### Example 4

To a mixture of sulfuric acid and chlorosulfonic acid, obtained by adding 2.8 ml of chlorosulfonic acid to 5.6 ml of 98% sulfuric acid, previously cooled on a ice bath and stirred until the development of foam ceased, 280 mg of hyaluronic acid sodium salt (HA-2) were added in small portions in about 20 minutes, then the mixture was let to stand at 4°C for 90 minutes under stirring. At the end of the reaction the mixture was poured into 100 ml of ethyl ether cooled with liquid nitrogen and the solid was filtered, washed with ethyl ether thus obtaining a greenish powder which was taken up with water. The solution was neutralized with a saturated solution of sodium bicarbonate and concentrated to dryness with rotavapor to give, after desalting by dialysis against water with 2000-Da cut-off membranes 230 mg of depolymerized, oversulfated hyaluronate having an average molecular weight of 14,100 Da and a sulfation degree of 3.4-3.6 (SHA-4).

### Example 5

To verify the molecular weight dependence, the mixture of two oversulfated products SHA-3 (Mw 15,850) and SHA-4 (Mw 14,100) were put together to give sample SHA-5. Its ¹³C-NMR spectrum only shows minor signals of free glucosamine 4-OH (14%) and 6-OH (8%), glucuronic acid 2-OH (11%) for a degree of sulfation higher than 3.5 and lower than 4. It was fractionated by gel fractionation on 2.2 x 120 cm Ultrogel ACA44, column using NH₄Cl 0.1 M in water as eluent and UV detection at 210 nm.(flow 1.4 ml/minute). Three fractions having different molecular weights were obtained: (SHA-5 Fr1, SHA-5 Fr2, SHA-5 Fr3) having an average molecular weight of 28,600; 10,300; and 3,200 Da respectively.

In the test of heparanase inhibition described above, this oversulfated hyaluronate fractions gave a mean inhibition of 86.5, 81.5, and 15.8%, respectively, at a concentration of 1 µg/ml and of 94.9, 91.6, 42.5 %, respectively, at a concentration of 5 µg/ml against a 79.7% inhibition at 1 µg/ml and 81.3% at 5 µg/ml with heparin and 63.6% inhibition at 1 µg/ml and 83.4% at 5 µg/ml obtained with maltohexaose polysulfate.

### Example 6

To a mixture of sulfuric acid and chlorosulfonic acid, obtained by slowly adding 5 ml of chlorosulfonic acid to 10 ml of 98% sulfuric acid, previously cooled to 4°C on a ice bath and stirred until the development of foam ceased (about 45 minutes), 500 mg of hyaluronic acid sodium salt (HA-1) were added in one portion, the mixture was let to stand for 20 minutes under stirring were added in one portion, whereby the reaction mixture became dark brown, then the mixture was quickly poured into 100 ml of ethyl ether, previously cooled to -40°C by liquid nitrogen, then the solid was filtered and washed with ethyl ether cooled to at least -20°C. The green-black powder thus obtained was taken up with 0.1 M sodium hydroxide and neutralized

The oversulfated hyaluronate thus obtained was dissolved in 5 ml of water and fractionated by size exclusion fractionation on a 5 x 67 cm TSK 40S column using 10% ethanol in water as eluent and UV detection at 210 nm. (flow 5,4 ml/min), by monitoring the fractionation by UV at 210 nm. Four fractions (SHA-6 Fr1; SHA-6 Fr2; SHA-6 Fr3; SHA-6 Fr4) having an average molecular weight of 86500; 25000; 22900; 33200 dalton respectively), each consisting of 100 ml of solution, were separated. The two first fractions were collected and submitted to a further fractionation.

The third fraction (SHA-6 Fr3) was concentrated to dryness to give an oversulfated hyaluronate fraction having a sulfation degree higher than 3.6, practically 100% 6-sulfated on glucosamine, 83% 4-sulfated on glucosamine, 83% 3-sulfated on glucuronic acid and 90% 2-sulfated on glucuronic acid.

In the test of heparanase inhibition described above, repeated twice at a concentration of 1 µg/ml and once at a concentration of 5 µg/ml, this oversulfated hyaluronate fraction gave a mean inhibition of 90.2% at a concentration of 1 µg/ml and of 94.1% at a concentration of 5 µg/ml, against a 79.7% inhibition at 1 µg/ml and 81.3% at 5 µg/ml obtained with heparin and 63.6% inhibition at 1 µg/ml and 83.4% at 5 µg/ml obtained with maltohexaose polysulfate.

The solution made of the two previously collected fractions (SHA-6 Fr1; SHA-6 Fr2) was poured onto a 2.5 x 150 cm Sephadex G25 column and fractionated using 10% ethanol in water as eluent with monitoring at the UV at 210 nm. Three fractions, called SHA-7 Fr1, Fr2 and Fr3 were separated. Fraction SHA-7 Fr2 was concentrated to dryness to give an oversulfated hyaluronate fraction having an average molecular weight of 27400 Da, a sulfation degree of 3.6, 93% 6-sulfated on glucosamine, 80% 4-sulfated on glucosamine, practically more than 90% 3-sulfated and more than 80% 2-sulfated on glucuronic acid.

In the test of heparanase inhibition described above, repeated once at a concentration of 1 µg/ml, this oversulfated hyaluronate fraction gave a mean inhibition of 89.8% at a concentration of 1 µg/ml and of 92.3% at a concentration of 5 µg/ml, against a 79.7% inhibition at 1 µg/ml and 81.3% at 5 µg/ml obtained with heparin and 63.6% inhibition at 1 µg/ml and 83.4% at 5 µg/ml obtained with maltohexaose polysulfate.

### Example 7

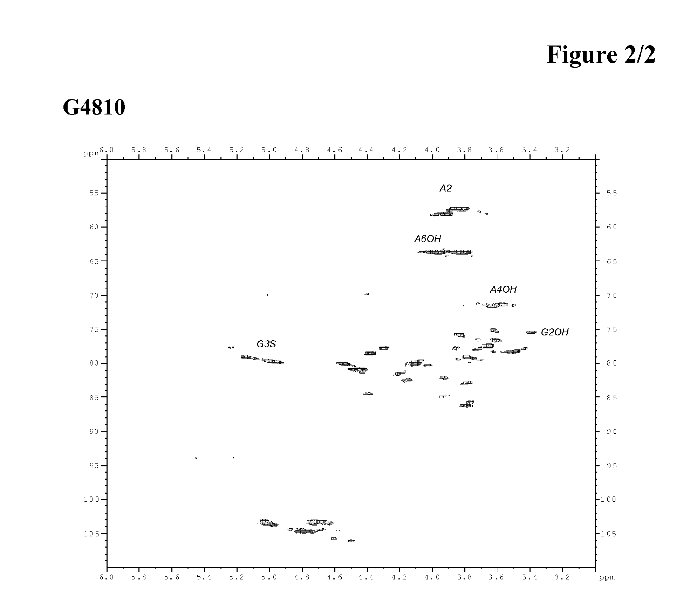

A solution of 200 mg of the depolymerized, oversulfated hyaluronate of Example 1 in about 2 ml water was poured onto a column filled with Amberlite IRA-120 H⁺ (regenerated with 4% hydrochloric acid). The eluate was neutralized with pyridine and concentrated with rotavapor to dryness. The obtained pyridinium salt of the depolymerized, oversulfated hyaluronate was suspended in 20 ml of dry pyridine. To the suspension thus obtained, heated at 60°C, 4.78 ml of bis-(trimethylsilyl)acetamide were added to the obtained suspension and the mixture was let to stand 2 hours under stirring at 60°C. At the end of the reaction 20 ml of water were added to the mixture, which was then neutralized with sodium hydroxide and concentrated to dryness to give, after desalting by size exclusion fractionation on a 5 x 67 cm TSK 40S column using 10% ethanol in water as eluent and UV detection at 210 nm. (flow 5,4 ml/min), 154 mg of 6-O-desulfated hyaluronate sulfate having an average molecular weight of 26,000 (SHA-8), consisting of a mixture of chains showing the repetitive sequence of formula I wherein R₆ is hydrogen in 83% of said chains, R₄ is hydrogen in 70% and SO₃⁻ in 30% of said chains, R₃ is hydrogen in 60% and SO₃⁻ in 40% of said chains and R₂ is hydrogen in 4% and SO₃⁻ in 96% of said chains, for a degree of sulfation of 1.8 (Figure 2).

In the test of heparanase inhibition described above (three experiments), this 6-desulfated hyaluronate gave a mean inhibition of 55.0% at a concentration of 1 µg/ml and of 86.4.8% at a concentration of 5 µg/ml, against a 79.7% inhibition at 1 µg/ml and 81.3% at 5 µg/ml obtained with heparin and 63.6% inhibition at 1 µg/ml and 83.4% at 5 µg/ml obtained with maltohexaose polysulfate.

### Comparative Example 8

To a mixture of sulfuric acid and chlorosulfonic acid, obtained by adding 2 ml of chlorosulfonic acid to 4 ml of 98% sulfuric acid, previously cooled on a ice bath and stirred until the development of foam ceased, 200 mg of hyaluronic acid sodium salt having a mean molecular weight of 187.000 Da (HA-2) were added in small portions in about 10 minutes, then the mixture was let to stand at 4°C for 90 minutes under stirring. After this time interval, the mixture was let to stand for 60 minutes at 25°C. At the end of the reaction the mixture was poured into 100 ml of ethyl ether cooled with liquid nitrogen and the solid was filtered, washed with ethyl ether thus obtaining a greenish powder which was taken up with a NaHCO₃ saturated solution. The solution was concentrated to dryness with rotavapor to give, after desalting by size exclusion fractionation on a 5 x 67 cm TSK 40S column using 10% ethanol in water as eluent and UV detection at 210 nm. (flow 5,4 ml/min), 28.6 mg of depolymerized, oversulfated hyaluronate having an average molecular weight of 1,916 Da (SHA-9). Its sulfation pattern, determined according to Guerrini et al. Analytical Biochemistry 337 (2005) 35-47 on HSQC NMR spectrum, Fig. 1(b), shows signals of 6-OH glucosamine (29%), major signals of free 4-OH glucosamine (60 %), and non-sulfate glucuronic acid (67%), for a degree of sulfation of ∼1.5.

The results obtained according to this example show that treatment of hyaluronic acid with a sulfuric acid/chlorosulfonic acid, under the conditions described in EP 116251 for other polysaccharides, does not give any oversulfation and affords very low molecular products in low yields.

## Claims

1. A process for the preparation of a glycosaminoglycan consisting of sulfated hyaluronate fractions and/or fragments having an average molecular weight of from 6,000 to 40,000 Da, which comprises
(a) reacting hyaluronic acid with a sulfuric acid/chlorosulfonic acid mixture at a temperature of from -15°C to +5°C and optionally submitting the product thus obtained to a size-fractionation, whereby O-oversulfated hyaluronate fractions and/or fragments with an average molecular weight of from 8,000 to 38,000 Da are obtained, consisting of a mixture of chains in which each chain in said mixture of chains shows the repetitive sequence of formula I 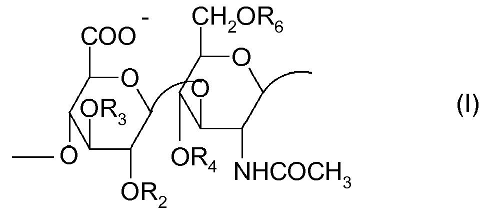 wherein each of R₂, R₃, R₄ and R₆ represents hydrogen or a SO₃⁻ group for a sulfation degree of from 2.8 to 4.0; and, optionally,
(b) treating the tertiary amine or quaternary ammonium salt of the oversulfated hyaluronate fractions and/or fragments mixture thus obtained with N,O-bis-(trimethylsilyl)acetamide, or N-methyl-N-(trimethylsilyl)trifluoroacetamide at 45-110°C, whereby sulfated hyaluronate fractions and/or fragments with an average molecular weight of from 6,000 to 30,000 Da, consisting of a mixture of chains in which at least 90% of said chains show the repetitive sequence of formula I' 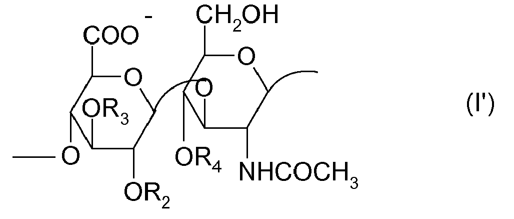 wherein each of R₂, R₃ and R₄ represents hydrogen or a SO₃⁻ group, for a sulfation degree of from 1.8 to 3, are obtained.

2. The process of claim 1, wherein the sulfated hyaluronate fractions and/or fragments mixture obtained at the end of each of steps (a) and (b) are isolated as sodium salt which is optionally converted into another salt.

3. The process of claim 2, wherein, other salts is selected from the group consiting of potassium, calcium, magnesium, pyridinium, tetra(*n*-butyl)ammonium, aluminum and copper salts.

4. A glycosaminoglycan obtainable according to the process of claims 1-3.

5. Sulfated hyaluronate fractions and/or fragments according to claim 4, having a molecular weight of form 6,000 to 38,000 and a sulfation degree of from 1.8 to 4.0.

6. A glycosaminoglycan according to claim 4, having an average molecular weight of from 8,000 to 38,000 Da, consisting of a mixture of chains of different length in which each chain in said mixture of chains shows the repetitive sequence of formula I 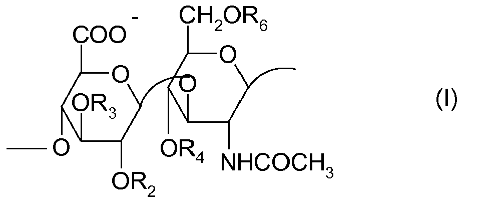 wherein each of R₂, R₃, R₄ and R₆ represents hydrogen or a SO₃⁻ group, for a sulfation degree of from 2.8 to 4.0 and the corresponding cation is a pharmaceutically acceptable one.

7. The glycosaminoglycan of claim 4, having an average molecular weight of from 6,000 to 30,000 and consisting of a mixture of chains in which at least 90% of said chains show the repetitive sequence of formula I' 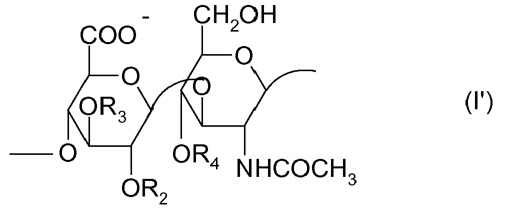 wherein each of R₂, R₃ and R₄ represents hydrogen or a SO₃⁻ group, for a sulfation degree of from 1.8 to 3 and the corresponding cation is a pharmaceutically acceptable one.

8. A pharmaceutical composition comprising, as an active ingredient thereof, a glycosaminoglycan according to anyone of claims 4 to 7, in admixture with a pharmaceutical carrier.

9. The composition of claim 8, as antimetastatic and tumor growth inhibiting agent.

10. Use of the glycosaminoglycans of anyone of claims 4 to 7, for the preparation of medicaments with antimetastatic and tumor growth inhibiting activity.
